# EUROPEAN PATENT SPECIFICATION

(11) **EP 3 581 094 B1**
(45) Date of publication and mention of the grant of the patent: **16.03.2022**
(21) Application number: 18211872.9
(22) Date of filing: 12.12.2018
(51) Int. Cl.: A61B 5/00

(54) **A SYSTEM FOR ATTACHING A MEASURING PROBE TO PROVIDE MONITORING OF TRANSPLANTED ORGANS**
SYSTEM FÜR BEFESTIGUNG DES MESSFÜHLERS ZUR ÜBERWACHUNG DER VERPFLANZTEN ORGANE
UN SYSTEME DE FIXATION D'UNE SONDE POUR SURVEILLER DES ORGANES GREFFES

(30) Priority: 14.06.2018 CZ 2018292
(43) Date of publication of application: 18.12.2019
(73) Proprietor: Ceské vysoké ucení technické v Praze, 16000 Praha 6- Dejvice (CZ)
(72) Inventor: BORTEL, Radoslav, 96001 Zvolen (SK); HOSPODKA, Jiri, 27341 Brandysek (CZ); SEBEK, Jan, 16000 Praha 6 (CZ); JANOUSEK, Libor, 14800 Praha 4 - Kunratice (CZ); MALY, Stepan, 10900 Praha 10 - Petrovice (CZ)
(74) Representative: Kratochvil, Vaclav

(56) References cited:
- WO-A1-2014/140283
- US-A- 4 926 875
- US-A1- 2012 053 396

## Description

### Background of the Invention

The presented solution deals with implants for monitoring the function of transplanted organs.

### Description of Prior Art

After an organ is transplanted, there is increased risk of its failure, for example because the blood inflow or outflow may become blocked. For early diagnostics, it is vital to continuously monitor an organ's condition, which may be done for instance by means of sensory implants placed directly onto an organ itself, see for example Kyriacou, P. A., M. Hickey, and J. P. Phillips. "Pulse oximetry of body cavities and organs." Engineering in Medicine and Biology Society (EMBC), 2013 35th Annual International Conference of the IEEE. IEEE, 2013. Such implants provide useful information for several hours or days after a transplantation. After this period, the risk that the transplanted organ would fail decreases and it is desirable to remove the measuring probe from the patient's body. This removal should be done without surgery, ideally simply by pulling the measuring probe out from the patient's body.

The requirement for easy removal, however, is in contrary to the requirements for quality measurement of the markers indicating the function of a transplanted organ. Many implants use the principles of pulse oximetry or they measure the blood flow rate based on the Doppler effect and the quality of such measurements may be negatively affected by the measuring probe movement against the monitored organ. Such movements may be eliminated by fixing the probe by surgical sutures, but this will not allow for easy removal of the probe when needed.

To facilitate probe extraction from the patient's body several solutions have been presented, for example those described in document US4926875A and related WO1989006513A1, WO1992021284A1 and related US5205292, US5160338A, US6346080B1. The common feature of all these solutions is the use of a probe body in the form of a flexible strip, which is made of a biocompatible material and which is attached by surgical sutures. This attachment is temporary, employing various uses of an extractible fixing wire which, when removed, releases the sutures, thus allowing easy removal of the probe from the patient's body. These solutions facilitate the probe extraction; however, they have numerous setbacks. Some solutions, such as US6346080B1, US5205292, require suturing the probe to the monitored organ, which harms the organ itself. Other solutions, such as US4926875A, US5160338A, place the sutures only on the probe body and the sutures are then extracted along with the probe. The major setback of this solution, however, is that once the sutures are released, they form a free loop, which, when being extracted alongside the probe, may get stuck in the patient's body, cause injury, or make the probe removal out of the patient's body completely impossible. Suturing also complicates the application of the measuring probe - the surgeon has to position the probe to the place of its application and then make relevant sutures. Such action may be cumbersome in case the place of application is not easy to access. In addition, this increases a surgeon's workload and prolongs the surgery. Another issue is that all the mentioned solutions rely on bringing the fixing wire to the surgical sutures in a way that will allow its reliable extraction. This complicates the mechanical design of the probe - apart from the wire itself, a guiding duct has to be created, in which the wire can move freely. In addition, there is increased risk of unsuccessful extraction in cases when blood or body fluids ingress into the guiding duct where they may coagulate and make easy extraction of the fixing wire impossible. Another substantial risk is the existence of the guiding duct, which creates a way to possibly introduce infection into the patient's body.

Last but not least, although the solutions mentioned above allow the application onto an organ, they are primarily intended for monitoring the blood flow through blood vessels and they are not suitably designed to monitor the condition of organs.

### Summary of the Invention

The disadvantages mentioned above are removed by the system for attaching a measuring probe to provide monitoring of transplanted organs according to the presented solution. The measuring probe body is made of biocompatible material, it is strip-shaped and it consists of a sensory part, which accommodates the measuring sensors and is to be placed onto the monitored organ, and of an output part that is to be lead out of the patient's body.

The principle of the new solution is the following arrangement. The system is formed by at least two grips in a form of strips made of a soft and elastic biocompatible material. The first ends of the grips are fixed from one side to the sensory part of the measuring probe body, in which the coils of solenoids are placed; their number corresponds to the number of the grips. The power supply conductors of these coils lead through the output part outside the measuring probe body to the power supply source. The second ends of the grips are equipped with permanent magnets, which are to be placed against the corresponding solenoid coils.

The advantage of this arrangement is that the measuring probe can be easily applied onto the monitored organ where it does not require fixing by surgical suturing; therefore, there is no risk the sutures might get captured during the extraction of the measuring probe from the patient's body. Another advantage is the absence of a guiding duct, a fixing wire placed in it and all related mechanical actions. Body fluids cannot ingress into the guiding duct and possibly coagulate, which might block the fixing wire extraction and thus prevent the release of the measuring probe from the monitored organ and its subsequent extraction from the patient's body.

In addition, the risk of introducing any impurities into the patient's body through the guiding duct is eliminated. The presented solution is designed for monitoring of organs as opposed to many currently available solutions, which are designed for application on blood vessels.

### Explanation of Drawings

The measuring probe and the method of its attachment are described by means of drawings in Fig. 1 to 4:
Fig. 1 shows the measuring probe with the indicated positions of the solenoid coils and the permanent magnets.
Fig. 2 shows the measuring probe applied onto the monitored organ.
Fig. 3 shows the measuring probe after the grips are released.
Fig. 4 shows a detailed arrangement of the measuring probe where the second ends of grips are not fixed to the measuring probe body. This drawing shows in closer detail how the solenoid coils, permanent magnets, and power supply conductors are arranged.

### Detailed Description of the Preferred Embodiments

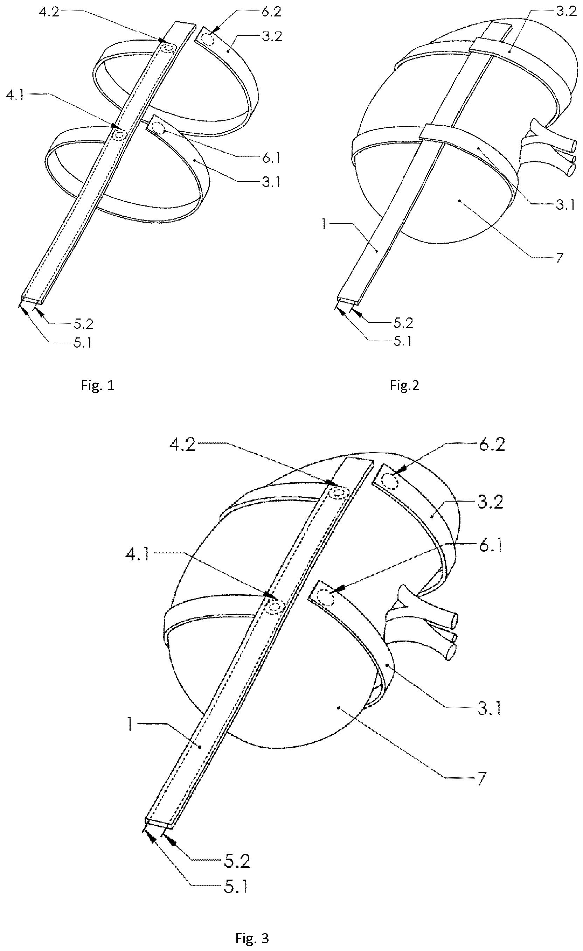

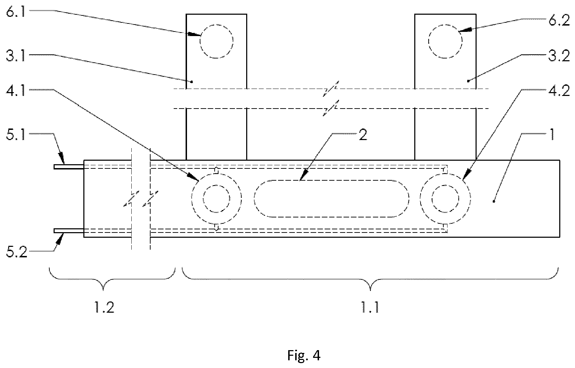

An example of the advantageous embodiment of the measuring probe is presented in Fig. 1 to 4. In this embodiment, a measuring probe body 1 is made of a soft biocompatible material, the probe carries sensors 2 for monitoring of the condition of an organ 7. The part of the measuring probe body 1, which carries the sensors 2, so-called sensory part 1.1, is intended to be placed onto the monitored organ 7, and the remaining part of the measuring probe body 1, so-called output part 1.2, leads out from the patient's body, Fig. 4. In the given example, two grips are attached to the measuring probe body 1, the first grip 3.1 and the second grip 3.2, which are made of a soft and elastic biocompatible material, such as silicone. The first end of each of the grips 3.1 and 3.2 is attached from one side to the measuring probe body 1. At the second end, both grips 3.1 and 3.2 hold a permanent magnet. The first grip 3.1 holds the first permanent magnet 6.1 and the second grip 3.2 holds the second permanent magnet 6.2. The measuring probe body 1 carries also the solenoid coils, the number of which corresponds to the number of permanent magnets, so in this case it means the first solenoid coil 4.1 and the second solenoid coil 4.2, which are powered by the first power supply conductor 5.1 and the second power supply conductor 5.2, which are lead through the measuring probe body 1 out from the patient's body. The current of the solenoid coils 4.1 and 4.2 can be supplied by a power supply source located outside the patient's body, not shown in the drawings. Advantageously, the power supply source may consist of a combination of a mains power supply, an electro-chemical accumulator, and a power supply control circuit, which will ensure an uninterrupted power supply for the solenoid coils even in the case when the mains power supply or the accumulator would disconnect.

The permanent magnets 6.1 and 6.2 allow to fix the second end of each of the grips 3.1 and 3.2 to the measuring probe body 1 in a detachable way by bringing them close to their relevant solenoid coils 4.1 and 4.2 and by connecting the power supply source to the power supply conductors 5.1 and 5.2 of the solenoid coils 4.1 and 4.2. Connecting the power supply will create a magnetic field and related forces, which will hold the permanent magnets 6.1 and 6.2 firmly at the second ends of the grips 3.1 and 3.2 against the measuring probe body 1. The second ends of the grips 3.1 and 3.2 may be released without any mechanical action, simply by disconnecting the power supply source from the power supply conductors 5.1 and 5.2 of the solenoid coils 4.1 and 4.2. When the power supply disconnects, the electromagnetic field disappears and the fixation of the second ends of the grips 3.1 and 3.2 to the measuring probe body 1 releases, which also releases the fixation of the measuring probe to the monitored organ 7, and the probe can be extracted from the patient's body.

When applying the measuring probe, it is placed onto the monitored organ 7 and the power supply source is connected to the power supply conductors 5.1 and 5.2. Subsequently, the permanent magnets 6.1 and 6.2 are placed against the solenoid coils 4.1 and 4.2, where they are held by the magnetic field force created by the solenoid coils 4.1 and 4.2, Fig. 2.

When extracting the measuring probe, the power supply source disconnects from the power supply conductors 5.1 and 5.2, which cuts off the current flowing through the solenoid coils 4.1 and 4.2. As a result, the force holding the permanent magnets 6.1 and 6.2 to the grips 3.1 and 3.2 disappears, and the measuring probe can be easily extracted from the patient's body, Fig. 3.

### Industrial Applicability

The presented solution can be used for time-limited monitoring of organs after transplantation surgeries.

## Claims

1. A system for attaching a measuring probe to provide monitoring of transplanted organs, the system comprising said measuring probe, where a measuring probe body (1) is strip-shaped and made of a soft biocompatible material, and it consists of a sensory part (1.1) to be placed onto a monitored organ (7), which accommodates measuring sensors (2), and of an output part (1.2) providing outputs from the patient's body **characterized by the fact that** the system for attaching the measuring probe body (1) to the monitored organ (7) is formed by at least two grips in a form of a strip, specifically by a first grip (3.1) and a second grip (3.2), which are made of a soft and elastic biocompatible material, while first ends of the grips (3.1, 3.2) are fixed from one side to the sensory part (1.1) of the measuring probe body (1), where a first solenoid coil (4.1) and a second solenoid coil (4.2) are placed, and where a first power supply conductor (5.1) and a second power supply conductor (5.2) of these coils are lead to the power supply source through the output part (1.2) outside the measuring probe body (1), while the second end of the first grip (3.1) is equipped with a first permanent magnet (6.1) to be placed against the first solenoid coil (4.1) and the second end of the second grip (3.2) is equipped with a second permanent magnet (6.2) to be placed against the second solenoid coil (4.2).

## Patentansprüche

1. System zum Anbringen einer Messsonde zur Überwachung von transplantierten Organen, das System umfasst eine Messsonde, wobei der Körper der Messsonde (1) streifenförmig und aus einem biokompatiblen Material besteht und aus einem sensorischen Teil (1.1) zur Platzierung auf einem überwachten Organ (7) besteht, wobei im sensorischen Teil zwei Messsensoren (2) angeordnet sind, und aus einem Ausgangsteil (1.2) zum Ausgang aus dem Körper des Patienten, **gekennzeichnet dadurch, dass** das System zum Anbringen des Körpers der Messsonde (1) an einem überwachten Organ (7) mindestens aus zwei streifenförmigen Griffen besteht, aus einem ersten Griff (3.1) und einem zweiten Griff (3.2), die aus einem weichen und flexiblen biokompatiblen Material bestehen, deren erste Ende an einer Seite am sensorischen Teil (1.1) des Körpers der Messsonde (1) befestigt ist, in der eine erste Magnetspule (4.1) und eine zweite Magnetspule (4.2) angeordnet ist, und wobei ein erster Versorgungsleiter (5.1) und ein zweiter Versorgungsleiter (5.2) dieser Magnetspulen an die Stromquelle durch einen Ausgangsteil (1.2) außerhalb des Körpers der Messsonde (1) geführt werden, wobei das zweite Ende des ersten Griffes (3.1) mit einem ersten Permanentmagneten (6.1) zum Platzieren über die erste Magnetspule (4.1) versehen ist und das zweite Ende des zweiten Griffes (3.2) mit einem zweiten Permanentmagneten (6.2) zum Platzieren über die zweite Magnetspule (4.2) versehen ist.

## Revendications

1. Système de fixation de sonde de mesure pour surveiller les organes transplantés, le système comprend une sonde de mesure où le corps (1) de la sonde de mesure se présente sous forme d'une bande en matériau biocompatible mou et est constitué d'une partie sensorielle (1.1) à placer sur l'organe surveillé (7) où, dans la partie sensorielle, des capteurs de mesure (2) sont placés, et d'une partie de sortie (1.2) à sortir à l'extérieur du corps du patient, **caractérisé en ce que** le système de fixation du corps (1) de la sonde de mesure à l'organe surveillé (7) est constitué d'au moins deux prises en forme de bandes, et ce, d'une première prise (3.1) et d'une deuxième prise (3.2) qui sont constituées d'un matériau biocompatible mou et flexible, dont la première extrémité est fixée d'un côté à la partie sensorielle (1.1) du corps (1) de la sonde de mesure dans laquelle une première bobine (4.1) ainsi qu'une deuxième bobine (4.2) de l' électroaimant sont placées et où le premier conducteur (5.1) d'alimentation et le deuxième conducteur (5.2) d'alimentation de ces bobines sont amenés à la source d'alimentation au moyen d'une partie de sortie (1.2) à l'extérieur du corps (1) de la sonde de mesure, la deuxième extrémité de la première prise (3.1) étant pourvue d'un premier aimant permanent (6.1) à placer au-dessus de la première bobine (4.1) de l'électro-aimant et la deuxième extrémité de la deuxième prise (3.2) étant pourvue d'un deuxième aimant permanent (6.2) à placer au-dessus de la deuxième bobine (4.2) de l'électro-aimant.
